# EUROPEAN PATENT SPECIFICATION

(11) **EP 2 528 581 B1**
(45) Date of publication and mention of the grant of the patent: **06.11.2019**
(21) Application number: 11710273.1
(22) Date of filing: 27.01.2011
(51) Int. Cl.: A61K 8/44, A61K 8/81, A61K 8/39, A61K 8/891, A61Q 19/00, A61K 8/58, A61K 8/92

(54) **METHOD AND COMPOSITIONS FOR THE APPLICATION OF AMINO ACIDS ON THE SKIN THROUGH AN ANHYDROUS MEAN**
VERFAHREN UND ZUBEREITUNGEN ZUR ANWENDUNG VON AMINOSÄUREN AUF DIE HAUT MIT EINEM WASSERFREIEN MITTEL
PROCÉDÉ ET COMPOSITIONS POUR L'APPLICATION D'ACIDES AMINÉS SUR LA PEAU PAR L'INTERMÉDIAIRE D'UN COMPOSITE ANHYDRE

(30) Priority: 27.01.2010 IT FE20100002
(43) Date of publication of application: 05.12.2012
(73) Proprietor: Ambrosialab S.r.L., 44121 Ferrara (IT)
(72) Inventor: MANFREDINI, Stefano, I-44121 Ferrara (IT); VERTUANI, Silvia, I-44121 Ferrara (IT)
(74) Representative: Feltrinelli, Secondo Andrea
(86) International application number: PCT/IB2011/000133
(87) International publication number: WO 2011/092581

(56) References cited:
- JP-A- 2002 037 714
- US-A1- 2005 008 598
- US-A1- 2008 319 069

## Description

A method based on amino acids for the prevention and/or dermatologic and/or cosmetic treatment of the skin disorders and of the related cutaneous structures, in particular nails and hair, for use in applications for moisturising, accelerating wound healing times, anti-age efficiency, antibacterial action, lightening/illuminating action, treating herpes and psoriasis, antioxidant and detoxifying action, barrier effect. In such method, an amino acid and/or a derivative and/or a mixture is carried in a hydrophobic medium to improve efficiency, stability and tolerability of the product.

### PRIOR ART

Amino acids are essential compounds for the metabolism of the skin and important components of the natural moisturising factor (NMF) in the horny layer.

The latter is a mixture that serves to maintain the correct amount of humidity, preventing excess demoisturising of the keratin lamellae. In this way, it allows keeping the surface of the skin whole, elastic and flexible.

NMF is formed by various substances and it is generated, besides keratin, by the cell breakage. Actually, when the cells of the skin die and form the horny layer, the proteins present therein are degraded into amino acids which constitute almost half of the NMF.

This mixture, fundamental for maintaining a correct equilibrium in the skin, has the disadvantage of being water soluble, hence it is easily eliminated by water and detergents during washing.

An important objective of modern functional cosmetics is that of reconstructing this natural layer of moisturizing substance on the skin surface. The use of amino acids in the field of dermatology and cosmetics has been known over time. Actually, these molecules have emollient, cicatrizing and restitutive properties and they are used in creams and lotions with moisturising, anti-wrinkles action etc.

It is known that each amino acid has specific properties and performs particular actions on the skin.

Some of the main actions of the amino acids which have the effect of protecting and keeping the skin healthy are indicated below by way of example:
- L-Lysine: conditioning agent for the skin and for repairing tissues, in that required for the formation of collagen. It confers the skin a smooth velvety effect. It is efficient at reducing symptoms caused by Herpes Simplex infection
- L-Histidine: it has antioxidant properties and protects the skin against the harmful effects of UV radiations
- L-Arginine: it accelerates the wound healing times
- L-aspartic acid: involved in the formation of DNA
- L-Threonine: important for the formation of collagen and elastin
- L-Serine: involved in the formation of cellular membranes
- L-Proline: important for keeping the structures of the skin healthy and functional
- L-Glycine: crucial for repairing tissue damages, it enhances wound healing
- L-Alanine: involved in protein synthesis
- L-Methionine: it has antioxidant and detoxifying properties
- L-Isoleucine: a branched chain amino acid, it plays an important role in protein synthesis and in tissue repair
- L-Cysteine: it is one of the main amino acids of keratin, to which it confers stability and resistance due to the formation of disulfide bonds. It contributes to reinforcing and keeping the structures of the skin and the related cutaneous structures (nails and hair) functional
- L-Leucine: a branched chain amino acid, serves a protection action against protein degradation
- L-Tyrosine: a fundamental protein constituent, it helps the penetration of moisturising agents into the skin. Furthermore, it is converted by the cells of the skin into melanin, which protects against the harmful effects of UV radiations. It can be used as L-Acetyl-tyrosine, the corresponding bioavailable form
- L-Phenylalanine: an amino acid present in numerous proteins, it can be converted into L-Tyrosine
- Taurine: it is a natural aminosulfonic acid which stimulates the synthesis of all the three classes of barrier lipids (ceramides, cholesterol, fatty acids) in the skin. Furthermore, it accelerates reparation of skin damages caused by heat stimuli or UV radiations.

Due to the properties thereof, the amino acids are widely used in the cosmetic industry for preparing formulations for maintaining a healthy skin and for the prevention and treatment of imperfections or mild disorders. Given that amino acids are water soluble molecules, the commercial products containing them are generally emulsions (creams, lotions, etc.).

Emulsions are oil dispersions in water or vice versa and thus subject of instability. Furthermore, given that they contain water, they create problems related to risks of contamination and thus they require addition of emulsifiers, stabilisers and preservatives which complicate the formulation of the product, increase the number of ingredients, the costs, the risk of intolerance, allergies and toxicity.

The object of the present invention is to provide a rational solution to the problem through vehicles with extremely low water content and where solubilisation of amino acids in a hydrophobic medium is obtained through fat-soluble chelating agents.

### DESCRIPTION OF THE INVENTION

The present invention regards a cosmetic formulation for topical use containing at least one amino acid in a mainly hydrophobic vehicle for the treatment of skin and the related cutaneous structures, in particular nails and hair, as a novel approach for solving numerous dermatologic and/or cosmetic problems.

In particular, the subject of the invention is a composition as claimed in claim 1 for dermatologic and/or cosmetic use as a hydrophobic gel, oleolite or butter comprising at least one amino acid; at least one hydrophobic vehicle such as oil, silicone or butter; a porous polymer thickeners and stabilisers of the hydrophobic phase; hydrogenated oils, silicone and non-silicon waxes such as bee wax; hydrophobic silicas. The amount of amino acids used may widely vary from 0.01% to 30%, preferably from 0.05% to 10%, even more preferably from 0.1% to 5% of the total weight of the cosmetic formulation. The amino acids, alone or combined, are selected from among L-alanine, L-aspartic acid, L-glutaminic acid, L-glycine, L-proline, L-serine, L-tyrosine, L-cysteine, L-arginine, L-histidine, L-leucine, L-isoleucine, L-lysine, L-methionine, L-phenylalanine, L-threonine, L-tryptophan, L-valine, L-asparagine, L-glutamine, L-acetyl-tyrosine, taurine.

The hydrophobic composition constituting the vehicle for the amino acids comprises, in weight/weight percentage on the total weight of the formulation, from 70% to 90% of suitable oil, butter or silicone vehicle for cosmetic use; such as: vegetable oils like squalene, argania spinosa, adansonia digitata; synthetic oils such as decyl oleate, caprylic capric triglyceride.

The silicon medium is preferably selected from among the group including pentamer cyclomethicone, tetramer cyclomethicone, hexamer cyclomethicone, dimethicone, phenyl trimethicone, diphenyl trimethicone, polysilicone-11, hexamethyldisiloxane, disiloxane, dimethicone/vinyl dimethicone crosspolymer, dimethicone crosspolymer or a mixture thereof.

The formulation, according to the present invention, may also include another oil component at a percentage comprised between 5% and 20% selected from among vegetable oils and esters of fatty acids, such as for example octyl palmitate, isopropyl myristate, ethyl oleate, ethers, fatty alcohols, unsaponifiables, such as those derived from olive, avocado, soy oil or a mixture thereof.

Regardless of the mainly hydrophobic characteristic of the formulation, the composition contains small percentages of water (not exceeding 10%) in association to a porous polymer, which is a methylmethacrylate crosspolymer, in a polymer/water ratio comprised between 4:6 and 7:3. Said polymer facilitates the solubilisation of the active components in the formulation increasing stability thereof and simultaneously contributes to improve the likability of the preparation.

Either microspheres or cyclodextrins can be used in association therewith also with the aim of increasing stability, facilitating a slow release and increasing efficiency.

With the aim of improving the stability of the hydrophobic formula, such as for example a hydrophobic gel, the formulation may include aluminium/magnesium hydroxide stearate, or hydrophobic silicas such as silica dimethyl silylate, or polyethylene and derivatives or hydrogenated oils, such as castor oil or rheocin, or natural waxes such as bee wax, minerals such as ozokerite, and synthetic waxes such as silicone waxes. An example consists in hydrogenated castor oil or rheocin at percentage, with respect to the total weight of the composition, comprised between 1% and 5%.

The incorporation of the amino acids, substances soluble in water, in an anhydrous vehicle offers interesting advantages with respect to the conventional formulations (emulsions).

Firstly, the hydrophobic gel subject of the present invention contains a limited number of ingredients, such characteristic being particularly desired, given that the lower the number of components inserted in the cosmetic formulation the lower is the risk of developing sensitivity or allergy phenomena due to single allergens.

Furthermore, the hydrophobic vehicle described by the present invention is characterized by low water content. This offers the important advantage of increasing the stability of the composition with a lower risk of microbial contamination. In particular, the innovative combination of ingredients used in the present formulation is characterized by a surprising microbiological stability which thus allows eliminating preservatives entirely. It is actually known that preservatives used in the dermatology and/or cosmetic field are among the greatest sensitising agents present in products for topic use, responsible for unwanted adverse reaction, including contact dermatitis, urticaria, photosensitisation. Elimination of preservatives from the formulation allows safely extending the use even to sensitive and allergic subjects or subjects with sensitive skin.

The hydrophobic form whose example is a hydrophobic gel, offers the further advantage of making the product adapted to a packaging incompatible with water, such as for example the use of capsules made of plant or animal gelatine in single dose of like softgel. Furthermore, such formulation has distinctive characteristics of cosmetic likability, due to a silky and velvety texture and an excellent spreadability and flowability on the skin. These characteristics allow the product to be easily applied on the skin, where it forms a thin and homogeneous film which facilitates the penetration of the active ingredients.

The cosmetic composition subject of the invention is characterised by an ideal stability over time both from a microbiological evaluation and from a viscosity and rheological properties point of view. The formulation for topical use described in the present invention is indicated for use in methods for the treatment of the skin and the related cutaneous structures (nails and hair) in wide range of methods, comprising as a result for example moisturising, accelerations of wound healing times, anti-ageing effect (by promoting the synthesis of collagen and inhibition of collagenase which determines an increase of the collagen of type I), antibacterial action, lightening/illuminating action, dermatological and/or cosmetic treatment of herpes and psoriasis, antioxidant and detoxifying action, barrier effect.

### EXAMPLES

With the aim of further illustrating the present invention, following are two general examples, non-limiting of the present invention, describing a possible method for providing a hydrophobic form based on amino acids, such as a hydrophobic gel, according to what has been described up to now:

**Example 1**

| **Ingredients** | **% weight** |
|---|---|
| Cyclomethicone | 51 |
| Dimethicone Crosspolymer | 35 |
| Amino acid (or combination of several amino acids) | 2 |
| Methylmethacrilate Crosspolymer | 5 |
| Water | 5 |
| Rheocin | 2 |

Amino acids, or a mixture thereof, and water are mixed together up to obtaining a limpid solution. The methylmethacrylate crosspolymer is added thereto, stirring using a helical stirrer (400 rpm for 30 minutes). Upon obtaining a homogeneous paste, cyclomethicone is added continuing to stir for another 30 minutes, after which dimethicone crosspolymer and rheocin are added to the compound continuing stirring up to obtaining a homogeneous gel.

**Example 2 (not invention)**

| **Ingredients** | **% weight** |
|---|---|
| Cyclomethicone | 46 |
| Dimethicone Crosspolymer | 30 |
| Amino acid (or combination of several amino acids) | 0.1 |
| Sorbeth-2 hexaoleate | 20 |
| Water | 2 |
| Rheocin | 1.9 |

Amino acids, or a mixture thereof, and water are mixed together up to obtaining a limpid solution. After being heated to 60°, sorbeth-2 hexaoleate is added thereto. The compound is stirred using a helical stirrer for 30 minutes, after which, still stirring, first cyclomethicone and at the end dimethicone crosspolymer and rheocin are added, up to obtaining a homogeneous gel.

As observable from the above-indicated non-limiting examples, the application of this method allows considerably simplifying the formula reducing the costs of preparation as well as the number of ingredients.

## Claims

1. A formulation for topical, dermatologic and/or cosmetic use, in form of hydrophobic gel, oleolite or butter comprising:
at least one amino acid;
at least a hydrophobic vehicle such as oil, silicone or butter;
thickeners and stabilizers of hydrophobic phase, hydrogenated oils, silicone waxes and non-silicone waxes such as bee waxes, silica;
a quantity of water;
**characterized in that** said formulation comprises a porous polymer, wherein the porous polymer is methylmethacrylate crosspolymer, wherein either microspheres or cyclodextrins are included in association therewith **and in that** said quantity of water does not exceed 10%, whereby said formulation is microbiological stable and free of preservatives.

2. A formulation for topical, dermatologic and/or cosmetic use according to claim 1, comprising, by weight percentage of the total weight of the formulation, preferably:
- from 0.05% to 10% of at least one amino acid,
- from 70% to 90% of at least one hydrophobic vehicle,
- from 2% to 20% of the porous polymer and/or the microspheres or cyclodextrins,
- from 1% to 5% of aluminum/magnesium hydroxide stearate, hydrophobic silica, such as silica dimethyl silylate, or polyethylene and derivatives, or hydrogenated oils such as castor oil or rheocin, or natural waxes such as bee wax, minerals such ozokerite, and synthetic waxes such as silicone waxes.

3. A formulation for topical, dermatologic and/or cosmetic use according to any one of claims 1-2, in which said amino acids may be of natural or non-natural origin chosen preferably among L-alanine, L-aspartic acid, L-glutamic acid, L- glycine, L-proline L-serine, L-tyrosine, L-cysteine, L-arginine, L-histidine, L-leucine, L-isoleucine, L-lysine, L-methionine, L-phenylalanine, L-threonine, L-tryptophan, L-valine, L-asparagine, L-glutamine, L-Acetyl-tyrosine, taurine.

4. A formulation for topical, dermatologic and/or cosmetic use according to any one of claims 1-3, in which the amount of amino acids is between 0.1% and 5%.

5. A formulation for topical, dermatologic and/or cosmetic use according to any one of claims 1-4, in which said silicone for dermatologic and/or cosmetic use can be chosen from among pentamer cyclomethicone, tetramer cyclomethicone, hexamer cyclomethicone, dimethicone, phenyl trimethicone, diphenyl trimethicone, polysilicone-11, hexamethyldisiloxane, disiloxane, dimethicone/vinyl dimethicone crosspolymer, dimethicone crosspolymer or a mixture thereof.

6. A formulation for topical, dermatologic and/or cosmetic use according to any one of claim 1-5, containing a quantity of water not exceeding 2%.

7. A formulation for topical, dermatologic and/or cosmetic use according to any one of claims 1-6, further containing a quantity of an oily component between 5% and 20%, selected from vegetable oils, esters of fatty acids, ethers, fatty alcohols, unsaponifiables or a mixture thereof.

8. A formulation according to any one of claims 1-7, for use in a topical dermatologic treatment of damage of mucosa, skin and skin annexes, such are hair and nail, in a topical dermatologic treatment of accelerations of wound healing times, antibacterial action, treatment of herpes, psoriasis and atopic dermatitis, antioxidant and detoxifying action, barrier effect.

9. Topical cosmetic use of a formulation according to any one of claims 1-7, for an anti-ageing effect, a moisturising, lightening/illuminating action of the skin and skin annexes, such are hair and nail.

## Patentansprüche

1. Formulierung zur topischen, dermatologischen und/oder kosmetischen Verwendung in Form von hydrophobem Gel, Oleolith oder Butter, umfassend:
mindestens eine Aminosäure;
mindestens ein hydrophobes Vehikel wie Öl, Silikon oder Butter;
Verdickungsmittel und Stabilisatoren der hydrophoben Phase, hydrierte Öle, Silikonwachse und silikonfreie Wachse wie Bienenwachse, Kieselsäure;
eine Menge Wasser;
**dadurch gekennzeichnet, dass** die besagte Formulierung ein poröses Polymer umfasst, worin das poröse Polymer Methylmethacrylat-Kreuzpolymer ist, worin entweder Mikrosphären oder Cyclodextrine in Verbindung damit enthalten sind, und dadurch, dass die Menge an Wasser 10 % nicht übersteigt, wodurch die besagte Formulierung mikrobiologisch stabil und frei von Konservierungsmitteln ist.

2. Formulierung zur topischen, dermatologischen und/oder kosmetischen Verwendung nach Anspruch 1, umfassend, als Gewichtsprozent bezogen auf das Gesamtgewicht der Formulierung, vorzugsweise:
- 0,05 % bis 10 % mindestens einer Aminosäure,
- 70 % bis 90 % mindestens eines hydrophoben Vehikels,
- 2 % bis 20 % des porösen Polymers und/oder der Mikrosphären oder Cyclodextrine,
- 1 % bis 5 % Aluminium/Magnesiumhydroxidstearat, hydrophobe Kieselsäure, wie Kieselsäuredimethylsilylat oder Polyethylen und Derivate, oder hydrierte Öle wie Rizinusöl oder Rheocin, oder natürliche Wachse wie Bienenwachs, Mineralien wie Ozokerit und synthetische Wachse wie Silikonwachse.

3. Formulierung zur topischen, dermatologischen und/oder kosmetischen Verwendung nach irgendeinem der Ansprüche 1-2, worin die besagten Aminosäuren natürlichen oder nicht-natürlichen Ursprungs sein können, ausgewählt vorzugsweise unter L-Alanin, L-Asparaginsäure, L-Glutaminsäure, L-Glycin, L-Proline L-Serin, L-Tyrosin, L-Cystein, L-Arginin, L-Histidin, L-Leucin, L-Isoleucin, L-Lysin, L-Methionin, L-Phenylalanin, L-Threonin, L-Tryptophan, L-Valin, L-Asparagin, L-Glutamin, L-Acetyl-Tyrosin, Taurin.

4. Formulierung zur topischen, dermatologischen und/oder kosmetischen Verwendung nach irgendeinem der Ansprüche 1-3, worin die Menge der Aminosäuren zwischen 0,1 % und 5 % liegt.

5. Formulierung zur topischen, dermatologischen und/oder kosmetischen Verwendung nach irgendeinem der Ansprüche 1-4, worin das besagte Silikon zur dermatologischen und/oder kosmetischen Verwendung ausgewählt werden kann aus Pentamercyclomethicon, Tetramercyclomethicon, Hexamercyclomethicon, Dimethicon, Phenyltrimethicon, Diphenyltrimethicon, Polysilikon-11, Hexamethyldisiloxan, Disiloxan, Dimethicon/Vinyldimethicon-Kreuzpolymer, Dimethicon-Kreuzpolymer oder einem Gemisch davon.

6. Formulierung zur topischen, dermatologischen und/oder kosmetischen Verwendung nach irgendeinem der Ansprüche 1-5, enthaltend eine Wassermenge von nicht mehr als 2 %.

7. Formulierung zur topischen, dermatologischen und/oder kosmetischen Verwendung nach irgendeinem der Ansprüche 1-6, ferner enthaltend eine Menge einer öligen Komponente zwischen 5 % und 20 %, ausgewählt aus Pflanzenölen, Estern von Fettsäuren, Ethern, Fettalkoholen, unverseifbaren Bestandteilen oder einem Gemisch davon.

8. Formulierung nach irgendeinem der Ansprüche 1-7 zur Verwendung bei einer topischen dermatologischen Behandlung von Schädigungen der Schleimhaut, Haut und Hautanhangsgebilden wie Haare und Nägel, bei einer topischen dermatologischen Behandlung von Beschleunigungen der Wundheilungszeiten, antibakteriellen Wirkung, Behandlung von Herpes, Psoriasis und atopischer Dermitis, antioxidativen und entgiftenden
Wirkung, Barrierewirkung.

9. Topische kosmetische Verwendung einer Formulierung nach irgendeinem der Ansprüche 1-7, für einen Anti-Aging-Effekt, eine feuchtigkeitsspendende, aufhellende/strahlende Wirkung der Haut und der Hautanhangsgebilde wie Haare und Nägel.

## Revendications

1. Formulation pour une utilisation locale, dermatologique et/ou cosmétique, sous forme de gel hydrophobe, d'oléolite ou de beurre comprenant :
au moins un acide aminé ;
au moins un véhicule hydrophobe tel que l'huile, le silicone ou le beurre ;
des épaississants et des stabilisateurs de phase hydrophobe, des huiles hydrogénées, des cires de silicone et des cires sans silicone telles que les cires d'abeille, la silice ;
une quantité d'eau ;
**caractérisée en ce que** ladite formulation comprend un polymère poreux, dans lequel le polymère poreux est un polymère réticulé de méthacrylate de méthyle, dans lequel des microsphères ou des cyclodextrines sont incluses en association avec celui-ci et **en ce que** ladite quantité d'eau ne dépasse pas 10 %, moyennant quoi ladite formulation est stable microbiologiquement et exempte de conservateurs.

2. Formulation pour une utilisation locale, dermatologique et/ou cosmétique selon la revendication 1, comprenant, en pourcentage de poids du poids total de la formulation, de préférence :
- de 0,05 % à 10 % d'au moins un acide aminé,
- de 70 % à 90 % d'au moins un véhicule hydrophobe,
- de 2 % à 20 % du polymère poreux et/ou des microsphères ou des cyclodextrines,
- de 1 % à 5 % de stéarate d'hydroxyde d'aluminium/magnésium, silice hydrophobe, telle que le silica dimethyl silylate, ou polyéthylène et dérivés, ou huiles hydrogénées telles que l'huile de ricin ou la rheocin, ou des cires naturelles telles que la cire d'abeille, des minéraux tels que l'ozocérite, et des cires synthétiques telles que les cires de silicone.

3. Formulation pour une utilisation locale, dermatologique et/ou cosmétique selon l'une quelconque des revendications 1-2, dans laquelle lesdits acides aminés peuvent être d'origine naturelle ou non naturelle sélectionnés de préférence parmi L-alanine, L-acide aspartique, L-acide glutamique, L-glycine, L-proline L-sérine, L-tyrosine, L-cystéine, L-arginine, L-histidine, L-leucine, L-isoleucine, L-lysine, L-méthionine, L-phénylalanine, L-thréonine, L-tryptophane, L-valine, L-asparagine, L-glutamine, L-acétyle-tyrosine, taurine.

4. Formulation pour une utilisation locale, dermatologique et/ou cosmétique selon l'une quelconque des revendications 1 à 3, dans laquelle la quantité d'acides aminés est comprise entre 0,1 % et 5 %.

5. Formulation pour une utilisation locale, dermatologique et/ou cosmétique selon l'une quelconque des revendications 1 à 4, dans laquelle ledit silicone pour une utilisation dermatologique et/ou cosmétique peut être sélectionné parmi le cyclométhicone pentamère, le cyclométhicone tétramère, le cyclométhicone hexamère, le diméthicone, le phényl triméthicone, le diphényl triméthicone, le polysilicone-11, l'hexaméthyldisiloxane, le disiloxane, le polymère réticulé de diméthicone/vinyl diméthicone, le polymère réticulé de diméthicone ou un mélange de ceux-ci.

6. Formulation pour une utilisation locale, dermatologique et/ou cosmétique selon l'une quelconque des revendications 1 à 5, contenant une quantité d'eau ne dépassant pas 2 %.

7. Formulation pour une utilisation locale, dermatologique et/ou cosmétique selon l'une quelconque des revendications 1 à 6, contenant également une quantité de composant huileux entre 5 % et 20 %, sélectionné parmi les huiles végétales, les esters d'acides gras, les éthers, les alcools gras, les insaponifiables ou un mélange de ceux-ci.

8. Formulation selon l'une quelconque des revendications 1 à 7, pour une utilisation dans un traitement dermatologique local des dommages liés à la muqueuse, la peau et les annexes de la peau, telles que les cheveux et les ongles, dans un traitement dermatologique local des accélérations de la durée de cicatrisation des plaies, de l'action antibactérienne, du traitement de l'herpès, du psoriasis et de la dermatite atopique, de l'action antioxydante et détoxifiante, de l'effet barrière.

9. Utilisation cosmétique locale d'une formulation selon l'une quelconque des revendications 1 à 7, pour un effet anti-âge, une action hydratante, éclaircissante/éclairante de la peau et des annexes de la peau, telles que les cheveux et les ongles.
